(19) Europäisches Patentamt
European Patent Office
Office européen des brevets

(11) **EP 1 390 750 B1**

(12) **EUROPEAN PATENT SPECIFICATION**

(45) Date of publication and mention
of the grant of the patent:
**09.03.2011 Bulletin 2011/10**

(21) Application number: **02728841.4**

(22) Date of filing: **19.04.2002**

(51) Int Cl.:
*G01N 33/53* (2006.01)      *G01N 33/543* (2006.01)
*G01N 33/537* (2006.01)

(86) International application number:
**PCT/US2002/012329**

(87) International publication number:
**WO 2002/085185 (31.10.2002 Gazette 2002/44)**

(54) **HYDROPHILIC DIAGNOSTIC DEVICES FOR USE IN THE ASSAYING OF BIOLOGICAL FLUIDS**

HYDROPHILE DIAGNOSEVORRICHTUNGEN ZUR VERWENDUNG BEIM TESTEN BIOLOGISCHER FLÜSSIGKEITEN

DISPOSITIFS DIAGNOSTIQUES HYDROPHILES UTILISES DANS L'ANALYSE DE FLUIDES BIOLOGIQUES

(84) Designated Contracting States:
**DE FR GB IE IT**

(30) Priority: **19.04.2001 US 284527 P**

(43) Date of publication of application:
**25.02.2004 Bulletin 2004/09**

(60) Divisional application:
**10158529.7 / 2 214 015**

(73) Proprietor: **ADHESIVES RESEARCH, INC.**
**Glen Rock,**
**PA 17327 (US)**

(72) Inventors:
• **MEATHREL, William, G.**
**York, PA 17404 (US)**
• **HAND, Herbert, M., Sr.**
**Bel Air, MD 21014 (US)**
• **SU, Li-Hung**
**Loganville, PA 17342 (US)**

(74) Representative: **Burford, Anthony Frederick**
**Beck Greener**
**Fulwood House**
**12 Fulwood Place**
**London**
**WC1V 6HR (GB)**

(56) References cited:
| EP-A- 0 408 378 | WO-A-99/27364 |
| DE-A- 3 903 126 | FR-A- 2 670 292 |
| GB-A- 2 341 924 | US-A- 3 960 499 |
| US-A- 5 904 824 | US-A- 5 962 215 |
| US-A- 5 985 675 | US-A- 6 087 185 |
| US-A- 6 150 180 | US-B1- 6 258 548 |
| US-B1- 6 284 194 | US-B1- 6 413 782 |

**Description**

**Background of the Invention**

This application is directed to novel hydrophilic constructions for in-vitro diagnostic test devices.

Lateral flow test strips are routinely used in medical and other applications to provide convenient and simple analysis of many important chemicals. S.M. Rosen, "Biomarkers of chemical exposure: A new Frontier in Clinical Chemistry", IVD Technology, May (1996) p.22; RA. Esposito, A.T. Culliford, S.B. Colvin et al., "The Role of the Activated Clotting Time in Herparin Administration and Neutralization for Cardiopulmonary Bypass", J. Thor. Card. Surg. 85 (1983), 174-185; C.A. McDonald, P. Syribeys, B. Hazelton, P. Bethea, T. Rigl, S. Hydo, S.J. Kennedy, 93rd General Meeting of American Society Microbiology, "A rapid 1-step colored particle lateral flow immunoassay for the detection of Group 1 Streptococcal Antigen extracted directly from Throat Swats", 93 (1993), p.507; and C. Huang and E. Fan, "One Step Immunochromatographic Device and Method of Use", U.S. Patent No. 5,712,172; A. Pronovost and J. Pawlak, "One Step Urine Creatine Assays", U.S. Patent No. 5,804,452.

Microtiter plates are used in the handling of liquid material samples during analytical assays for multiple, low volume analysis. Such plates involve the use of an assay plate having multiple depressions or wells, which provide a rapid automated analysis. Typically, such wells have a capacity of 1 microliter. Such microliter plates have a variety of uses, including enzyme assays, receptor-ligand assays, or cell based assays. The use of such microliter plates may be either batch-wise, or continuous.

The use of a continuous strip of material having sample wells molded along the length of the strip of material is disclosed in US. Patent No. 4,883,642. This patent discloses means to automatically hold, process, store and analyze biological samples comprised of a ribbon provided with microwells for analysis of multiple samples. The microwells in the ribbon may be protected by an adhered protective film or skin.

Microfluidic devices are also commonly-used in the assaying of biological samples. Such devices comprise a base platform within which are formed a number of capillaries which serve to transport the sample from a receiving portion of the device to a collection portion.

All of the above diagnostic devices are well-known to those skilled in the art.

In-vitro diagnostic devices are used to detect analytes such as nutrients, hormones, therapeutic drugs, drugs-of-abuse and environmental contaminates. In medical diagnostic test devices, biological fluids such as whole blood, plasma, serum, nasal secretions, sputum, saliva, urine, sweat, transdermal exudates or cerebrospinal fluids may be analyzed for specific components that are clinically important for monitoring and diagnosis. In addition, microbiological suspensions and tissues may be homogenized in compatible liquids and the fluid analyzed for specific components. Typically, the specimen fluid is deposited at an inlet port of a suitable in-vitro diagnostic test strip and the sample fluid is drawn into the device by mechanical means such as vacuum or by capillary flow action.

In-vitro diagnostic devices are used in various settings including hospitals, clinics, alternative care sites and in the home. These devices have been developed by various manufacturers to enable clinical professionals and non-professionals to make accurate decisions for the diagnosis and management of medical conditions. Point-of-care devices such are used to analyze blood chemistry such as electrolytes and pH in both clinical and non-clinical locations. Home pregnancy test kits are used to monitor hcG in urine. Diabetics routinely use diagnostic test strips to monitor blood glucose concentrations. Amira Medical, "Glucose Monitor without Fingersticking", IVD Technology, July 1999, p. 16.

A number of U.S. and foreign patents describe the use of lateral flow assay devices. U.S. Patent No. 5,798,27 and corresponding *European patent* 833159 describe a direct read lateral flow device for detecting small analytes. WO 97/38126 describes a lateral flow device for measuring analytes in whole blood. U.S. Patent No. 5,804,452 describes a device for the detection of creatinine in biological fluids such as urine in a one step lateral flow sensor. U.S. Patent No. 5,916,521 describes a vertical flow diagnostic device for the testing of body fluids. WO 99/34191 describes a lateral flow test strip for the detection of an analyte such as beta lactam in milk. See also, U.S. Patent Nos. 4,857,453*; 5,087,556; 5,137,808; 5,712,170; 5,712,172; 5,804,452; 5,821,073; 5,985,675; 5,989,921; 6,087,175 and 6,103,536.*

Various types of capillary flow type diagnostic devices are also known and have been used for some time. Exemplary of such devices are those shown in U.S. Patent Nos. 6,048,498 *and* 6,117,395.

**Objects and Summary of the Invention**

It is an object of the present invention to provide diagnostic devices which enable benefits to be achieved not achieved by prior art devices.

It is an object of the present invention to provide lateral flow devices which provide faster and more uniform flow of the sample, uniform wicking of membranes and a uniform capture line.

In accordance with the above, there is provided a lateral flow in-vitro diagnostic device comprising a housing, means in the housing to introduce a sample to be assayed in the device, means in the housing for fluid collection, and a backing strip having spaced apart first and second ends, wherein the surface of the backing strip is hydrophilic in character.

**Brief Description of the Drawings**

Figure la is a top view of a prior art lateral flow device.
Figure 1b is a schematic diagram of the prior art lateral flow device of Figure 1a;
Figure 2 is a depiction of capillary rise in a cylinder;
Figure 3 is a depiction of the wetting of a fluid on a smooth flat surface;
Figure 4 depicts a laboratory coating technique for casting adhesive on a film;
Figure 5 depicts a method for contact angle measurement on a flat surface;
Figure 6 depicts a micro fluidic device used in in-vitro sample analysis;
Figure 7 depicts the effect of surfactant concentration on contact angle;
Figure 8 depicts water contact angle vs. spreading time for hydrophilic films;
Figure 9 depicts water contact angle vs. surfactant concentration for films;
Figure 10 depicts the effect of surfactant concentration on contact angle and flow rate;
Figure 11 is a side view of a lateral flow diagnostic device of the present invention;
Figure 12 is a top view of the lateral flow diagnostic device of Figure 11;
Figure 13 is an exploded view of a lateral flow diagnostic test strip of the present invention; and
Figure 14 is an exploded view of another embodiment of a lateral flow test strip of the present invention;

**Detailed Description of the Invention**

In accordance with the present invention, adhesives and polymer films may be formulated using polymer resins and surfactants to provide multifunctional bonding properties for use in in-vitro diagnostic devices.

Hydrophilic adhesives or films may be formulated to be thermally bonded or pressure sensitive. The hydrophilicity of the surface of the adhesive or film is controllable through the chemical structure, concentration and distribution of the surfactant in the adhesive coating. The hydrophilic properties reduce the surface tension of biological fluids (e.g., blood, urine, and sputum), thus allowing the rapid transfer of fluid from an inlet area to a remote reagent area in an in-vitro diagnostic device.

The invention will be described in connection with the Figures.

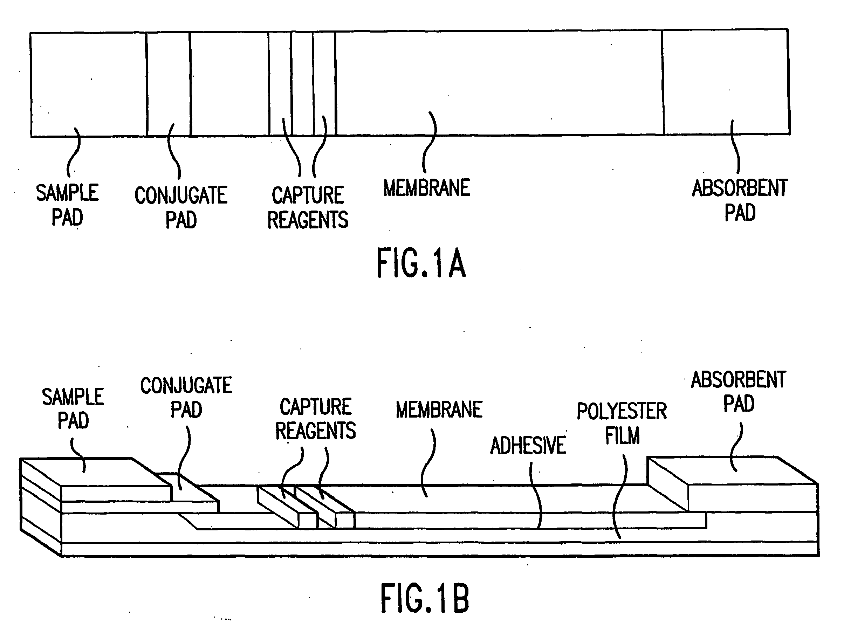

Lateral flow devices as shown in Figure 1 typically have a sample inlet area for receiving the biological fluid. The sample inlet area or port may be proximal to a conjugate pad that holds reagents specific to the analytical test method. As the sample specimen flows from the inlet area through a reagent area, specific chemical reactions or a complex formation occur. The reaction product or complex continues to flow to a detection area where the analyte is monitored. Specimen fluids may continue to flow and be collected in an absorbent pad. The time required for determining the concentration of a specific analyte is dependent on the flow rate of the fluid and the reaction rate between the analyte and a specific test reagent.

Adhesive backings are typically used in the construction of lateral flow devices to support the various components of the device including the conjugate pad, a microporous membrane with specific reagents and an absorbent pad as shown in Figure 1. The adhesive layer may be either pressure sensitive or heat-sealable, and may be present on a backing film such as a polyester film. The flow rate of the sample fluid is typically controlled by capillary flow through the microporous membrane.

Membranes used in lateral flow devices are typically hydrophobic polymers with low surface energy. These membranes are polymers such as nitrocellulose, nylon, polyether sulfone or polyvinylidiene. Consequently, these components are not compatible with aqueous biological fluids. To overcome the low surface energy of the membrane, surface active agents such as sodium dodecylsulfate (SDS) and sodium dodecylbenzene sulfonate (SDBS) are added to increase the wettability and consequent wicking ability of the membrane. Although the addition of surface active agents to the membrane increases its wettability these chemicals decrease the ability of the membrane to bond or retain proteins which may be critical to the analytical requirements and device performance. In addition, surfactants added to the membrane can reduce test sensitivity by reducing signal intensity due to extensive spreading of reagent bands.

It is known that the use of adhesives in diagnostic devices such as lateral flow devices to bond the hydrophilic membrane to the backing layer can result in a reduction in the effectiveness of the hydrophilic membrane layer during transport of the sample to be assayed across the membrane layer. Jones et al, IVD Technology, pp. 57-63, September, 2000. This reduced efficiency can be attributed to the migration of the adhesive into the membrane layer, creating isolated hydrophobic areas within the hydrophilic membrane. This effect is particularly enhanced upon use of an adhesive exhibiting low hardness (which exhibits high cold flow properties) in combination with a hydrophilic membrane of minimal thickness, thus enhancing the ability of the adhesive to affect the surface properties of the membrane. This effect can be minimized by the use of high hardness adhesives which exhibit low cold flow properties. However, high hardness adhesives also exhibit undesirable lower initial bond strength than a low hardness adhesive, a factor that must be taken into account when constructing the lateral flow device.

Strong intermolecular attractive forces exist between molecules to create surface tension. N. Vallespi i Salvado et al, "Surfactants in Pressure Sensitive Adhesives", Surface Coatings International, 4,1999, pp. 181-185. These intermolecular forces create high surface tension in aqueous biological fluids such as blood, urine, and sputum. In comparison, the surface energy of solid substrates is low. This differential between the surface tension of biological fluid and substrates commonly used to make in-vitro diagnostic devices needs to be overcome to achieve lateral flow and wicking.

Two approaches can be used to improve the flow of biological fluids through a diagnostic device. One approach is to increase the surface energy of the substrate (or membrane) with various surface treatments. A second approach is to reduce the surface tension of the biological fluid.

Adhesives are typically hydrophobic polymers with a surface energy ranging from 30 to 40 dyne cm$^{-1}$. An approach to increase the flow properties of in-vitro diagnostic devices is to increase the surface energy of the hydrophobic adhesive coating. There are a number of patents that describe the synthesis and utility of hydrophilic polymers and adhesives.

For example, U.S Patent No. 3,686,355 describes a block copolymer of a base polymer with a second surface modifying additive. U.S. Patent Nos. 5,354,815 and 5,614,598 describe polymers having enhanced hydrophilicity and thermal regulated properties. In this area, a hydrophilic polysiloxane anionic polymer is bonded to an aliphatic polyamide or polyester polymer fiber to enhance the hydrophilic and thermal properties of the textile. A number of U.S. and foreign patents are directed to the use of hydrophilic polymers used to formulate pressure sensitive adhesives. See, for example, U.S. Patent No. 5,508,313 (hydrophilic pendant moieties on polymer backbone), U.S. Patent No. 5,660,178 (hydrophilic crosslinking agents), U.S. Patent No. 6,121,508 (lipophilic pressure sensitive adhesive with a surfactant for skin contact in biomedical electrodes), WO 00/56828 (use of hydrophilic ester monomers that are polymerized to produce a wet stick pressure sensitive adhesive), EP 869979B (preparation of hydrophilic pressure sensitive adhesive using polar monomers), U.S. Patent No. 5,685,758 (hot melt adhesive with improved wicking for application to non-woven fabric), WO 97/48779 (hydrophilic hot melt adhesive composition prepared by blending adhesive components with a surfactant), and U.S. Patent No. 6, 040, 048 (water removable pressure sensitive adhesive containing hydrophilic pendent groups).

Polymeric films have modified surface properties are well known and produced by many distinct methods. See, for example, U.S. Patent Nos. 2,502,841 (gaseous chlorine); 2,829,070 (halogen gas); 3,142,582 (acid bath); 3,326,742 (halogenated organic amine); 3,561,995 (reactive conditioning agent with metal ion); 3,843,617 (aqueous acidic solution); 3,968,309 (surfactant-containing curable coating); 4,190,689 (titanium dioxide treatment); 4,387,183 (grafting hydrophilic chains to polymer surface); 4,416,749 (irradiation and surface hydrolysis); 4,460,652 (grafted hydrophilic polymer coating); 4,595,632 (hydroxy-fluorocarbon graft surface treatment); 4,666,452 (surface modified by hydrogen sulfato groups); 5,273,812 (hydrophilic film of hydrophilic monomer together with surface active agent); 5,280,084 (surface modification with carboxyl, carbonyl and hydroxyl groups followed by reaction with heterocyclic compound); 5,332,625 (crosslinked polymer surface); 5,451,460 (coating of non-ionic, hydrophilic surfactant in binder); 5,503,897 (irradiation and alkalization of polymer surface).

The present invention includes the selection of multifunctional coatings, adhesives and films and their use in in-vitro diagnostic devices. Hydrophilic substrates or constructions can be hydrophilic heat seal coatings as well as pressure sensitive adhesive tapes. Pressure sensitive adhesive tapes facilitate device manufacturing and are integral to device performance. The combination of a pressure-sensitive or heat-sealable adhesive with hydrophilic properties to aid lateral flow and the wicking of biological fluids will prove beneficial to device manufacturers. Benefits will include increased flexibility in device design, increased wicking rates and consequently faster test results. Increased wicking consistency and potentially reduced sample volume are some of the advantages to be achieved through the use of hydrophilic pressure sensitive adhesives and heat-sealable coatings.

In view of the above, the objects of the present invention include providing adhesive coatings or films with controllable hydrophilicity to increase the surface energy of the fluid flow path to enhance the flow of biological fluids in in-vitro diagnostic devices, providing hydrophilic adhesives that bond components of the diagnostic device thereby facilitating a more efficient manufacturing process for production of the device, increasing the transfer rate of the sample fluid from an inlet port to distal reagents and therefore reducing the time for analysis, enabling smaller sample volumes by enabling more efficient transport of fluid to a sensing reagent, and reducing risk of chemical interference by providing a wicking surface that allows an increased separation between the sampling port and the test reagents.

Hydrophilic coatings or films formulated by mixing surfactants with a polymer resin enhance the wicking of biological fluids into or through an in-vitro diagnostic medical device. Polymer resins may be selected from film forming polymers with a suitable glass transition temperature to form a hydrophilic coating. Similar resins may be selected for heat sealable hydrophilic coatings. In addition, resins typically used as pressure sensitive adhesives may be formulated with surfactants to provide a hydrophilic pressure sensitive adhesive. These constructions are dual functional in that they may serve to bond the components of the diagnostic device together and also to create high energy surfaces which reduce the surface tension of the biological fluid. The reduced surface tension of the fluid allows rapid transfer of the fluid from an inlet area to a remote reagent area in an in-vitro diagnostic device. The rapid fluid spreading can reduce the time needed for analysis. Since a smaller sample volume is required due to effective fluid wicking, device design flexibility is enhanced. This permits more efficient manufacturing processing with the potential for reduced product cost.

Hydrophilic coatings, films and adhesives can also be employed which do not require the incorporation of the surfactant into the formulation to provide the necessary hydrophilic properties. Examples of hydrophilic coatings and adhesives include polymers that can be cross-linked using di-hydroxyl terminated polyethylene glycol or polypropylene glycol monomers such as polyethylene glycol 600 supplied by Union Carbide Corporation. In addition, a vinyl terminated monomer with a hydrophilic moiety such as an anionic group can be grafted onto a polymer backing to increase the hydrophilic properties of the backing. One monomer that can be used is sodium AMPS, which is the sodium salt of 2-acrylamide-2-methyl-propanesulfonic acid, supplied by Lubrizol, which can be grafted onto the surface of a polymer by use of UV radiation. Such hydrophilic coatings can be used with and without the addition of a surfactant to provide a hydrophilic coating or adhesive.

Surface tension of a fluid is the energy parallel to the surface that opposes extending the surface. Surface tension and surface energy are often used interchangeably. Surface energy is the energy required to wet a surface. To achieve optimum wicking, wetting and spreading, the surface tension of a fluid is decreased and is less than the surface energy of the surface to be wetted. The wicking movement of a biological fluid through the channels of a diagnostic device occurs via capillary flow. Capillary flow depends on cohesion forces between liquid molecules and forces of adhesion between liquid and walls of channel. The Young/Laplace Equation states that fluids will rise in a channel or column until the pressure differential between the weight of the fluid and the forces pushing it through channel are equal. Walter J. Moore, Physical Chemistry 3rd edition, Prentice-Hall, 1962, p. 730.

$$\Delta p = (2\gamma \cos\theta)/r$$

where $\Delta p$ is the pressure differential across the surface, $\gamma$ is the surface tension of the liquid, $\theta$ is the contact angle between the liquid and the walls of the channel and r is the radius of the cylinder. If the capillary rise is h and $\rho$ is the density of the liquid then the weight of the liquid in the column is $\pi r^2 gh\rho$ or the force per unit area balancing the pressure difference is ghp.

Therefore $(2\gamma \cos\theta)/r = ghp$ or $h = 2\gamma \cos\theta/g\rho$. For maximum flow through membranes (fluid wicking), the radius of the channel should be small, the contact angle $\theta$ should be small and $\gamma$ the surface tension of the fluid should be large.

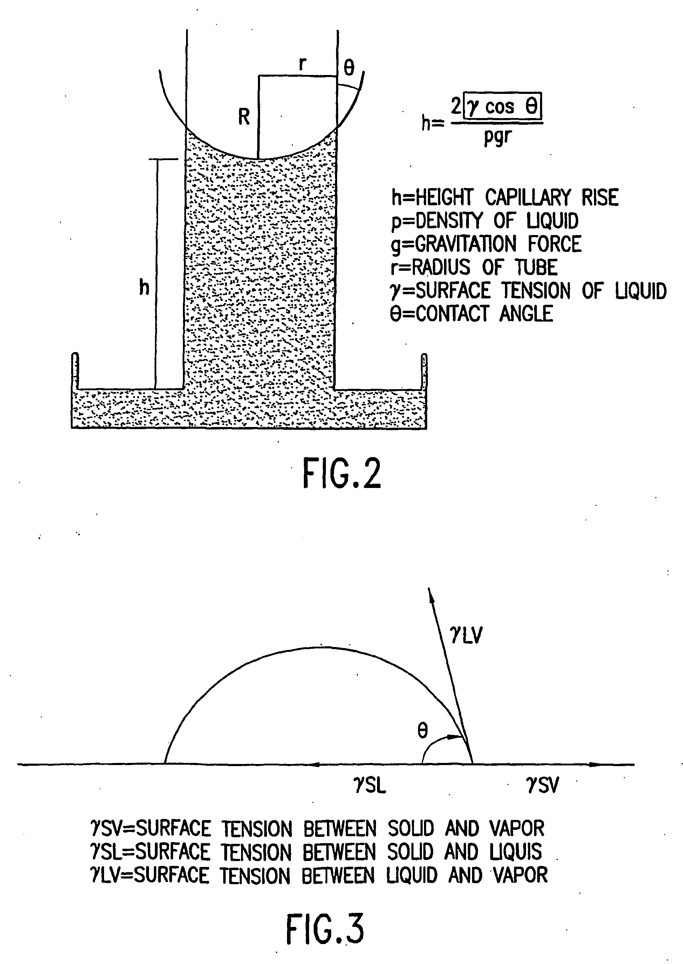

Wetting is the adhesion on contact between a liquid and solid. W.A. Zisman, "Influence of Constitution on Adhesion", Handbook of Adhesives, 2nd edition, Van Nostrand Reinhold Co., 1977, p. 38. For maximum wetting, the surface tension of the liquid must be less than or equal to the surface tension of the solid surface. This is the critical wetting tension of the solid. Figure 3 illustrates surface wetting of a fluid on a flat smooth surface.

The theoretical explanation of this phenomenon can be described by the classical model know as Young's Equation. T. Young, Philos. Trans. Roy. Soc. London, 95 (1805) p. 65.

$$\gamma_{SV} = \gamma_{SL} + \gamma_{LV}\cos\theta \text{------------------Eq. 1}$$

The diagram shown in Figure 3, illustrates the relationship between the contact angle $\theta$ and surface tension of liquid $\gamma_{LV}$ and solid $\gamma_{SV}$. W.A. Zisman, ibid, pp. 33-64. When the contact angle $\theta$ between liquid and solid is zero or so close to 0, the liquid will spread over the solid.

The spontaneous process of wettability can also be derived from the differential between work of adhesion and cohesion by substitution of Dupre Equation below in Equation 2:

$$W_A - W_C = \gamma_{SV} + \gamma_{LV} - \gamma_{SL} - 2\gamma_{LV} = \gamma_{SV} - (\gamma_{LV} + \gamma_{SL})\text{-------------Eq. 2}$$

This equation implies that spontaneous spreading will occur if the work required separating the liquid-solid interface is greater than liquid separation itself. Therefore, Equation 2 can be further derived by introducing the initial spreading coefficient S defined by Harkins ("The Physical Chemistry of Surface Films", Reinhold, 1952) and shown in Equation 3 below:

$$S = W_A - W_C = \gamma_{SV} - (\gamma_{LV} + \gamma_{SL})\text{------------------}Eq.3$$

Since $\gamma_{SL}$ is relatively small in comparison with $\gamma_{LV}$, the initial spreading coefficient term becomes:

$$S = \gamma_{SV} - \gamma_{LV}\text{--------------------}Eq.4$$

Spreading is the movement of liquid across a solid surface. Contact angle is a measure of wettability. Spreading increases as the contact angle decreases until wetting is complete. Hence, the spreading will occur spontaneously when S is greater than zero, which also indicates that the surface tension of the solid must be greater than that of the liquid, as shown in Equation 4. From the initial spreading coefficient equation showed above (Eq. 4), the wettability will occur either by increasing surface tension of the solid or decreasing surface tension of liquid.

The use of surfactants to lower the surface tension of a fluid is well known. M.J. Rosen, Surfactant and Interfacial Phenomena John Wiley & Sons, New York, (1978); Th.F. Tadros, Surfactants, Academic Press, Inc. New York, (1984); A.C. Clark et al, "New and Improved Waterborne Systems", Adhesives Age, September (1999), 33-40.

The effect of surfactants in coatings and adhesives has been studied to determine their effect on wettability, fluid flow rate and adhesive properties. Each surfactant was formulated into a base adhesive at different concentrations. The water contact angle was measured to determine the effect of surfactant on reducing the surface tension of the water.

**TABLE 2**

| Physical Properties of Selected Surfactants | | | |
|---|---|---|---|
| Chemical Description | Structure | Charge Types | Mol. Wt. |
| Sodium 2-Ethylhexyl Sulfate | Branched | Anionic | 232 |
| Sodium Lauryl Sulfate | Linear | Anionic | 288 |
| Sodium Nonylphenol Ether Sulfate | Aromatic | Anionic | 498 |
| Nonylphenol Ethoxylate | Aromatic | Nonionic | 704 |
| Polyalkyeneoxide Modified Heptamethyltrisiloxane | Linear Siloxane | Nonionic | 600 |

Hydrophilic coatings and heat-sealing and pressure sensitive adhesives were prepared. Dissolution of polymeric resins occurred in organic solvents. Dissolution was followed by measurement of solution solids and viscosity over a period of several hours.

The surfactant was introduced into the liquid polymer mixture after dissolution of the resin. Gentle agitation for several minutes was sufficient to achieve homogeneity. Hydrophilic pressure-sensitive formulations were prepared by the introduction of a surfactant into liquid acrylic adhesive solutions and emulsions followed by gently mixing until dispersed or dissolved.

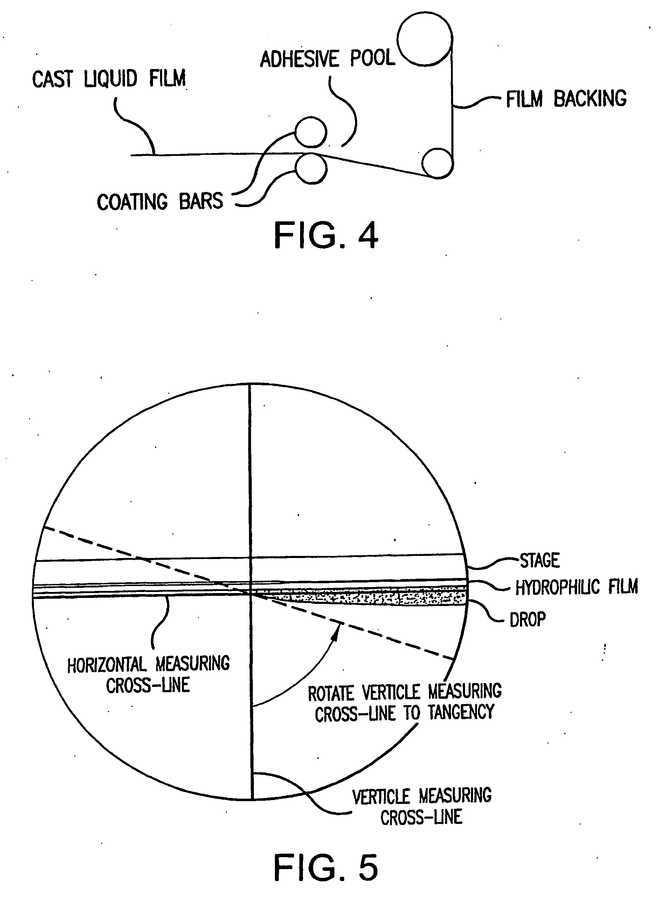

Hydrophilic films were prepared in the lab using coating apparatus. The lab preparations were accomplished by use of coating bars that evenly spread the liquid formulations on a film backing as shown in Figure 4. The liquid adhesive was first deposited as a pool onto a film backing, then the backing drawn through two stainless steel bars until the adhesive solution spread across and down the film to produce an even coating thickness. The thickness of the film was controlled the gap set between the two coating bars. The cast films were dried for five to ten minutes in a Blue M Stabil Therm convection oven set at 105°C. The dried coatings had an approximate thickness of 0.0005 to 0.001 inches (1.25 - 2.5 $\mu$m), as measured with a Mitutoyo Absolute Digital Thickness Gauge. The hydrophilic adhesive coatings were protected with a film substrate of low surface energy (release liner).

The hydrophilic coatings were tested for surface wetting using de-ionized water. The sessile drop method was employed to measure the contact angle liquid water makes with the surface of the hydrophilic thin film. A ramé hart contact angle goniometer was used.

A micropipette was used to draw deionized water from a beaker. Several drops of the liquid were dispensed back into the beaker to ensure a bubble free liquid. The micropipette was then mounted onto the goniometer.

An approximate 1" x 1" (2.5 x 2.5 cm) sample of hydrophilic film was place on the goniometer stage with the hydrophilic surface towards the drop. The film was flattened then secured to the stage by placing a magnet or clamps on each side of the film. Gloves were used when handling the film surface to avoid any oils or dirt from hands that could alter the surface of the film.

The micropipette was then lowered to just above the hydrophilic surface. A drop of water with a volume of approximately 2 $\mu$l was suspended on the tip and lowered towards the film until the water drop dispensed onto the surface. The drop of water was allowed to spread across the surface until equilibrium was established (30 seconds). The microscope was focused to view the extreme left or right of the resulting drop (see Figure 5). The cross-line inside the scope was adjusted to tangency above the base of the drop to create a wedge of light bounded by the two cross-lines and the drop profile. The cross-line was slowly rotated while adjusting the cross travel of the specimen stage assembly so that the wedge of light is gradually extinguished and the cross-line attains tangency with the drop profile at the base of the drop. The contact angle was read directly from the scope reticle at the six o'clock position. The contact angle was recorded to the nearest degree on both sides of the spread water drop.

Samples of the hydrophilic pressure sensitive and hydrophilic heat seal adhesive substrates were tested for peel adhesion to stainless steel panels: Testing was performed on a MTS Alliance RT/1 mechanical tester equipped with a 25-lb (11.3 kg) load cell and hydraulic grips. The machine was interfaced with a Dell Optiplex GX1p computer system containing MTS TestWorks software package and Hewlett Packard 895c printer.

The hydrophilic pressure sensitive adhesive tapes were tested for peel strength from 6" x 6" (15.25 x 15.25 cm) stainless steel panels using Adhesives Research ART 1005, "Five Minute Peel". The method is similar to ASTM D3330-83. The testing was carried out in a controlled temperature (70° F; 21°C) and humidity (50% RH) environment. Prior to testing, the stainless steel panels were cleaned with high purity urethane grade 2-butanone. The tape samples were cut to 1" x 10" (2.5 x 25 cm), then laminated (two passes) to the stainless steel panel using a 4.5-1b (2 kg), 80 durometer hardness roller. Peel testing was initiated after a five-minute dwell, by attachment of stainless steel plate to the bottom set of grips and overhanging, unbound portion of tape to the top set of grips. The tape was pulled away from the stainless steel plate at a rate of 12 inches (30 cm)/minute and at an angle of 180-degrees. The load and displacement were observed to increase to a maximum over the first 1-inch (2.5 cm) of the test then remain constant until the test was complete. The peel strength was calculated from the quotient of average load (oz) between one and five-inch (2.5 - 12.5 cm) displacement on the panel, and the sample width (in; cm).

Hydrophilic heat seal coatings were tested for adhesion using ASTM D1876-95. The testing was carried out at 70°F (21°C) and 50 % relative humidity. Adhesion was tested after lamination to a cleaned 7-mil (18 $\mu$m) polyester film. The samples were heat laminated on a Wabash press by exposure for 2 seconds at 100°C and at a pressure of 30-40 psi (200 - 275 kPa). The samples were aged four days at 70°F (21°C) and 50% R.H. Peel testing was conducted as described above and found to be acceptable.

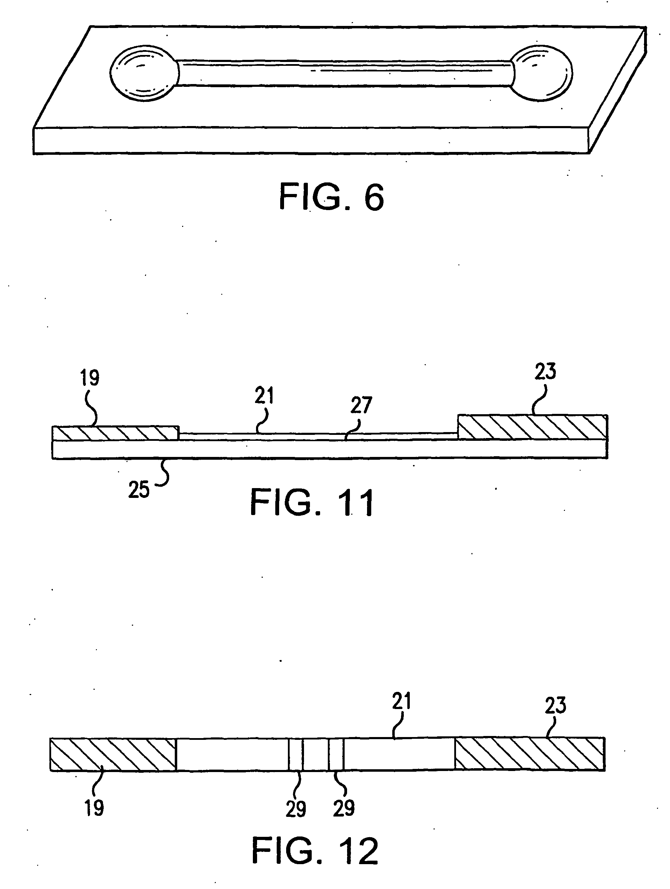

The effect of hydrophilic coatings and adhesives on the flow rate of distilled water in a microfluidic channel was investigated. Following a screening of the effect of different types of surfactants on contact angle, the most effective surfactants were formulated into adhesive tapes that were used as a cover for a microfluidic device as shown in Figure 6.

The microfluidic channel was molded in a device made from polystyrene. The channel had a length of 20 cm with a depth of 10 microns and a width of 30 microns. The hydrophilic tape was used to close the channel to create the microfluidic device. Distilled water was placed in one of the terminal wells and the time for the water to flow through the channel was measured.

Chemical surface analysis of the hydrophilic coatings was performed using infrared spectroscopy via attenuated total reflectance (ATR). The spectra were recorded using a Pike Miracle ATR single bounce sample port unit using a ZnSe crystal. Film samples were compressed onto the crystal using the compression arm at full contact pressure. Infrared spectra were collected using a MIDAC M1300 Series FT-IR bench with a mercury-cadmium-telluride (MCT) nitrogen cooled detector. Absorbance spectra were collected from 30 scans per sample from 4000cm$^{-1}$ to 600cm$^{-1}$ at 2 cm$^{-1}$ resolution at a gain of 1X. The FTIR bench was interfaced with YKE Microsystem computer and analyzed using Grams 32 software package.

The surface topography of the hydrophilic coatings was observed using atomic force microscopy. The instrument used was a Digital Instruments Nanoscope IIIa Multimode instrument. Hydrophilic tapes were mounted onto 1-cm diameter magnetic stubs and imaged in the tapping mode. Using this mode, the AFM cantilever is oscillated at its resonant frequency. Contact between the oscillating tip and the tape surface causes a decrease in the measured amplitude of oscillation. Since the contact is made at the largest displacement from the cantilever equilibrium position, little energy is transferred to the sample and minimal deformation of the sample occurs. Images were obtained by raster scanning the sample surface under the tip and recording the z motion of the sample necessary to maintain constant amplitude during the scan. This mode of imaging has several advantages over direct contact mode imaging. Lateral forces that are prevalent during contact mode scans are eliminated. Additionally, this tapping mode provides a non-destructive method for the imaging of soft samples. Importantly, phase images obtained using the tapping mode can give additional information concerning the mechanical and adhesive properties of the sample surface. A. Doring et al, "Atomic Force Microscopy: Micro- and Nano-Mapping of Adhesion, Tack and Viscosity", 23rd Annual Technical Seminar: Pressure Sensitive Adhesive Tapes for the New Millennium, May, 2000, pp. 213-222.

All samples were initially scanned in air. The hydrophilic coating HY-10 was then rinsed with de-ionized water for 10 seconds before being wiped dry with a paper tissue. The sample was left to dry overnight and imaged the next morning.

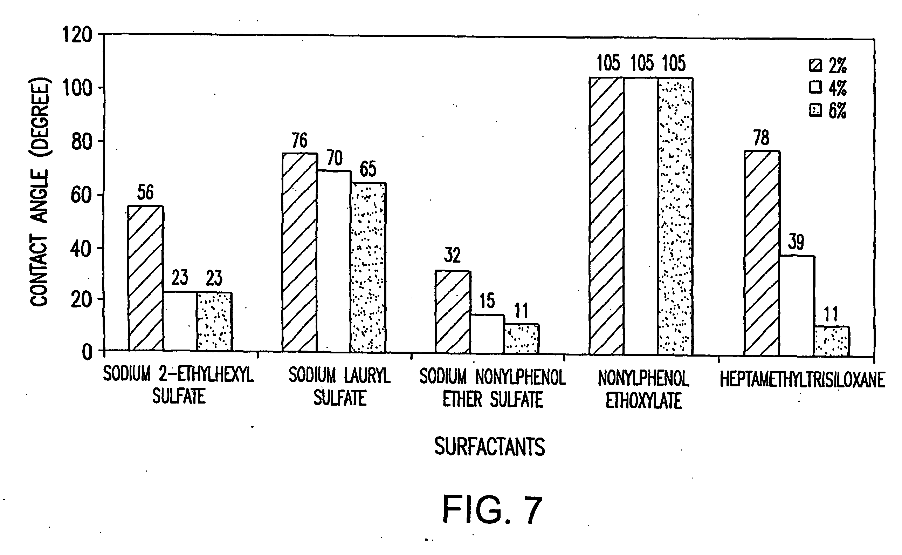

Various surfactants as shown in Table 2 were formulated into an emulsion pressure sensitive adhesive. Figure 7 shows that most test samples exhibit a similar trend of decreasing contact angle with increasing surfactant concentration. Sodium nonylphenol ether sulfate exhibited the most effective surface tension reduction of water at all three surfactant concentrations used in this study. The nonionic surfactant, nonylphenol ethoxylate, exhibited little effect on the contact angle of de-ionized water. This may be due to its higher molecular weight and the lower water affinity of the hydrophilic group compared to anionic type surfactants. In addition, the nonylphenol group enhances its absorption onto the polymer surface. Polyalkyeneoxide modified heptamethyltrisiloxane (PMHS) (SILWET™ L77 from Union Carbide), also a non-ionic surfactant, reduced the water contact angle of the adhesive surface compared with the nonylphenol ethoxylate. PMHS has a siloxane polymer backbone instead of a hydrocarbon backbone, which accounts for its lower surface energy. In addition, PMHS also has a lower molecular weight than nonylphenol ethoxylate which enhances its mobility within the adhesive matrix. PMHS can be formulated into a solvent-based pressure sensitive adhesive in amounts of up to 20% by wt. to increase the hydrophilic properties of the adhesive.

Of the surfactants evaluated, sodium nonylphenol ether sulfate had the highest molecular weight of the anionic surfactant used. It is believed that the lower molecular weight anionic surfactants have better solubility into the adhesive matrix so that the concentration of the surfactant at the water/adhesive interface is less. The linear structure of sodium lauryl sulfate may improve its solubility into the adhesive so that its effect on the adhesive surface is less than that of sodium 2-ethylhexyl sulfate.

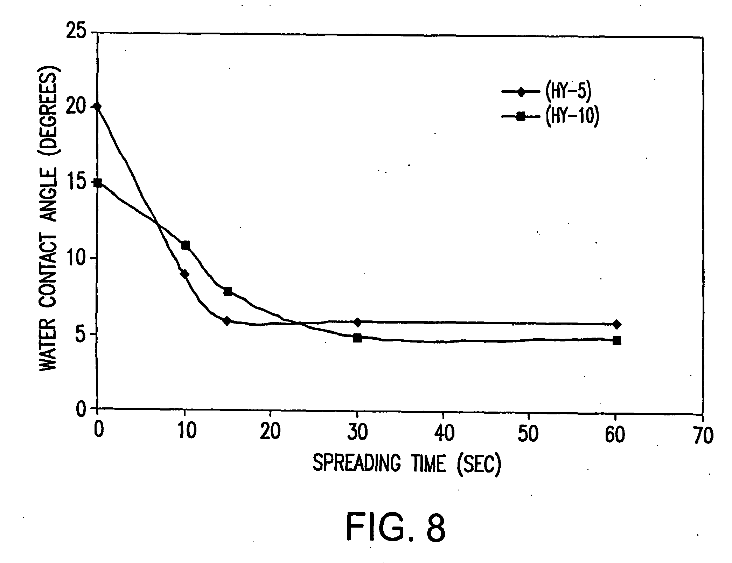

The wetting of the surface of HY-5 and HY-10 which are two hydrophilic heat seal adhesives was investigated by measuring the spreading of water. These hydrophilic heat seal adhesives were formulated using polyester resins and the anionic surfactants, sodium nonylphenol ether sulfate and sodium dioctylsulfo succinate, respectively. Figure 8 is a graph that describes the spreading behavior of water on the surface of HY-5 and HY-10 thin film coatings. Water was dropped onto the surface of the adhesives and the contact angle was measured as a function of time. Initially there is rapid spreading of the drop as it contacts the surface of the film. The contact angle decreases quickly to less than 10 degrees. Equilibrium is established within thirty seconds to one minute. This spreading behavior is typical of the hydrophilic coatings, heat-seal adhesives, and pressure sensitive adhesives.

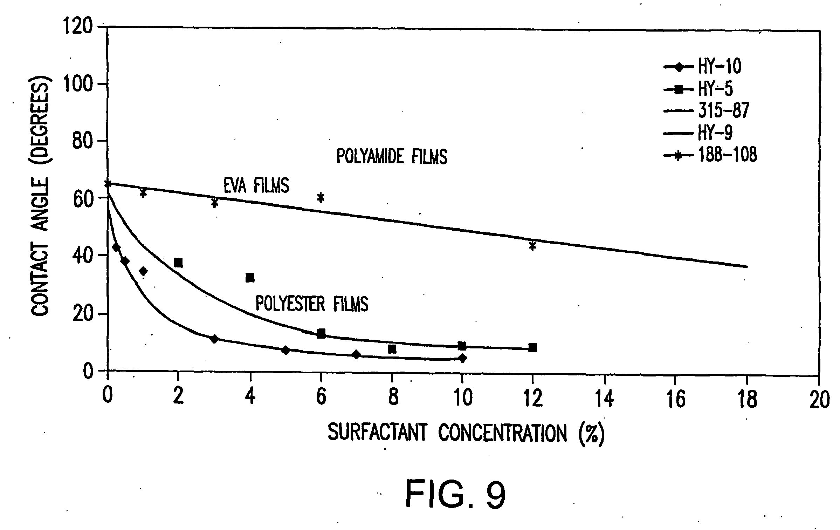

Figure 9 shows the effect of the surfactant concentration on the surface wettability of the dried films prepared using different polymeric resins. Polyamide, ethylene vinyl acetate, and polyester resins were formulated with sodium dioctylsulfo succinate. The resins studied included films of polyamide, ethylene vinyl acetate, and polyester chemistries. When no surfactant is present in the coatings the contact angle is high since the polymeric resins are hydrophobic. By increasing the surfactant concentration the surface becomes more hydrophilic and lower water contact angles are observed indicating significant surface wetting. At very high surfactant concentrations the wetting effect can be enhanced or attenuated depending on the surfactant and its compatibility with the polymer matrix.

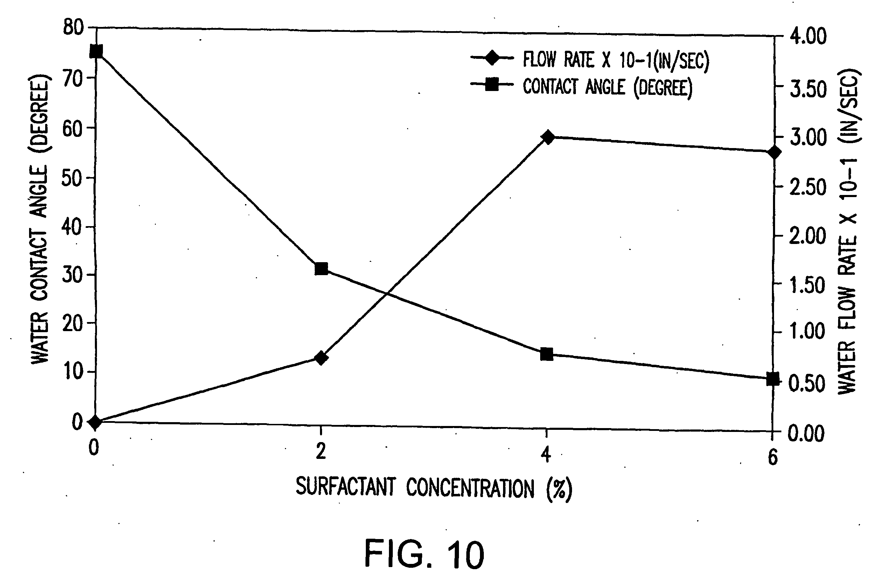

Figure 10 shows the effect of surfactant concentration on the rate of water flow in a covered microfluidic device (corresponding to device of Fig. 7). In this experiment, a hydrophilic pressure sensitive adhesive was formulated using concentrations of sodium nonylphenol ether sulfate ranging from 0 to 6 percent. When there was no surfactant added to the adhesive, water did not flow through the channel. With increasing concentration of surfactant the rate of water flow through the microchannels increased while the contact angle decreased.

The increased flow rate of water can be attributed to the reduction of water surface tension. The principle that could be used to explain this phenomenon is capillary rise as shown in Figure 2 which documents the relationship between the surface tension and the contact angle. The height of the water in the capillary is determined by a factor of two times the product of liquid surface $\gamma_{LV}$ and $\cos\theta$ regardless of liquid density and gravitation force. As a result, the water will advance further when the surface tension of water is close to the surface tension of the capillary material that is now determined by the hydrophilic adhesive cover. At high surfactant concentration (greater than 4% in Figure 10), the rate of flow levels off since the concentration of surfactant exceeds the critical micelle concentration. Additional surfactant on the surface of the adhesive does not reduce the surface tension of the fluid and may become autophobic. W.A. Zisman, "Influence of Constitution on Adhesion", Handbook of Adhesives, 2nd edition, 1977, p. 46.

Atomic force microscopy (AFM) was used to visualize the topography of a hydrophilic coatings. The AFM images of coatings containing 0%, 1%, 5% and 10% surfactant were obtained. The images show enrichment of the film surface at the film/air interface with increasing amount of surfactant introduced to the adhesive formula. The AFM image of the coating containing no surfactant shows a relatively smooth, flat surface.

Transformation is observed when 1% or less surfactant has been incorporated into the adhesive coating, where raised surface features are observed on the film surface. Increased surface topography is observed at 5% surfactant while at 10% surfactant the surface appears to be smoother due to saturation of the surface.

Infrared spectra of the coatings confirm the increase in surfactant concentration on the surface. The prominent peak at 2958 cm$^{-1}$ in the ATR is assigned to the C-H stretch of a $CH_3$ group on the surfactant in the hydrophilic adhesive and is used to monitor surfactant accumulation on the surface. A plot of absorbance of the C-H stretch as a function of concentration of surfactant at 0%, 1.0%, 5.0% and 10% shows a flattening resulting from the surface saturation by the surfactant

Hydrophilic coatings, hydrophilic pressure-sensitive and heat-sealable adhesives may be used in a variety of in-vitro diagnostic devices, including capillary flow, lateral flow, microfluidic, microtiter plates and electrophoretic devices.

The following are examples of various embodiments of the present invention:

**Example 1** (Hydrophilic Coating)

A polyester resin with a high glass transition temperature commercially available as Vitel™ 2200 BA from Bostik Chemical Company is dissolved in a solvent of methyl ethyl ketone and toluene (7:3 weight ratio). A commercial surfactant such as Rhodapex™ CO-436 available from Rhodia Inc. is dissolved in the resin solution to provide a surfactant solids to resin ratio of between 3:97 to 6:94. A hydrophilic coating is formed by spreading the resin/surfactant solution onto a polymer film and allowing the solvent to evaporate. Wetting the dried film surface with distilled water causes spreading of water on the surface. The contact angle of the water on the surface ranges from 5 to 10 degrees.

**Example 2** (Hydrophilic Heat Sealable Coating)

A similar formulation and coating as in Example 1 is prepared using a polyester resin with a lower glass transition temperature such as Vitel™ 3200 through 3500 series resins with a glass transition temperature between -15 °C to +15°C. One example is Vitel™ 3300B which has a Tg of+11°C. A heat sealable hydrophilic coating is formed by coating the formulation of resin and surfactant onto a surface such as a polymeric film and allowing the solvent to evaporate. The contact angle of the hydrophilic coating is similar to those of Example 1 (5 to 10 degrees). Other resins such as ethylene vinyl acetate and polyamide polymers may be used as heat sealable formulations.

**Examples 3-8** (Hydrophilic Pressure Sensitive Coatings - Aqueous Based)

A hydrophilic pressure sensitive coating is prepared by formulating an emulsion based resin such as Aroset™ 3500 available from Ashland Specialty Chemical Company (division of Ashland, Inc.), with a surfactant such as Rhodapex™ CO-433 available from Rhodia, Inc. The formulation was coated onto a hydrophobic polymer film such as 5 mil (12.5 μm) polyester film available from DuPont Teijin Films. After coating and drying the formulation, the contact angle was measured. The following table illustrates the effect of surfactant concentration on the contact angle which is related to the surface energy of the hydrophilic adhesive. The adhesive 180° peel force can be modified through addition of additives such as tackifiers.

| Acrylic Resin (%) Aqueous Solvent | Rhodapex™ CO-433 (%) | Contact Angle (Degrees) | 180° Peel (oz/inch) (g/cm) |
|---|---|---|---|
| 99 | 1 | 95 | |
| 98 | 2 | 32 | 68 (760) |
| 97 | 3 | 29 | |
| 96 | 4 | 15 | 6 (67) |
| 95 | 5 | 13 | |
| 94 | 6 | 11 | 7 (78) |

**Examples 9-12** (Hydrophilic Pressure Sensitive Coatings - Solvent Based)

Similar to Examples 3-8, hydrophilic pressure sensitive adhesive coatings are formulated using solvent based adhesives and surfactants. An acrylic resin adhesive in the organic solvent ethyl acetate was formulated with various concentrations of Rhodapex™ CO-433. After coating onto a polyester film and drying, the contact angle of the coating was measured. The following table shows the effect of surfactant concentration on the contact angle and 180° peel force.

| Acrylic Resin (%) Ethyl Acetate Solvent | Rhodapex™ CO-433 (%) | Contact angle (Degrees) |
|---|---|---|
| 97 | 3 | 33 |

(continued)

| Acrylic Resin (%) | Rhodapex™ CO-433 | Contact angle |
|---|---|---|
| Ethyl Acetate Solvent | (%) | (Degrees) |
| 94 | 6 | 17 |
| 91 | 9 | 16 |
| 88 | 12 | 15 |

In addition to the concentration of the surfactant, the surface energy of the hydrophilic coating can be controlled by the selection of surface active agent. The selection of surface active agent is based on factors such as molecular weight, linear vs. branched structure, ionic vs. non-ionic and the type of ionic moiety present, aromatic vs. aliphatic structure. These chemical structure properties can be used to control the hydrophilic characteristics and surface energy of the coating. The following table shows the effect on contact angle of 2 percent surfactant in Aroset™ 3500 coatings by the selection of surfactant structure. The following table shows the effect of surfactant characteristics on coating wettability:

| Surfactant/Contact Angle | Ionic Charge | Molecular Wt | Structure |
|---|---|---|---|
| Sodium 2- Ethylhexyl sulfate 41° | anionic | 232 | branched |
| Sodium octyl Sulfate 19° | anionic | 232 | linear |
| Sodium lauryl Sulfate 20° | anionic | 288 | linear |
| Sodium Nonylphenol sulfate 32° | anionic | 382 | aromatic |
| Nonylphenol Ethoxylate 105° | nonionic | 820 | aromatic |

A novel feature of using a hydrophilic coating formulated with Rhodapex™ CO-436 (the ammonium salt of sulfated nonylphenol ethoxylate) is the ability to pattern the surface energy of a uniform coating using radiant energy. When thermal energy is applied to the coating in a pattern such as stripes, circles or any other configuration, the surface energy in the area of applied energy is reduced. It is believed that ammonia gas is evolved due to the thermal energy leaving the sulfonic acid of nonylphenol ethoxylate remaining. The hydrophilicity of the coating decreases and consequently becomes water resistant. Radiant energy sources such as lasers and electron beam may also be employed to cause the evolution of a labile cation to customize the physical character of the coating. This may be used with advantage in the production of in-vitro diagnostic devices, such as by the application of thermal energy to the surface to produce a parallel, laterally-oriented, striped pattern of alternating hydrophilic/hydrophobic areas. The presence of the hydrophobic areas may be employed with advantage to slow the wicking of the material to be tested as it travels from a hydrophilic region to a hydrophobic region, whereby additional time for reaction between the analyte and the reagent results. Fluid wicking through the device may be retarded over areas of lower surface energy to permit time for reaction or complex formation by use of a single coating. This may be employed to avoid too rapid fluid wicking which may be detrimental if the reaction time is insufficient. Of course, a series of reaction zones of various shapes and configurations can be created on a single film.

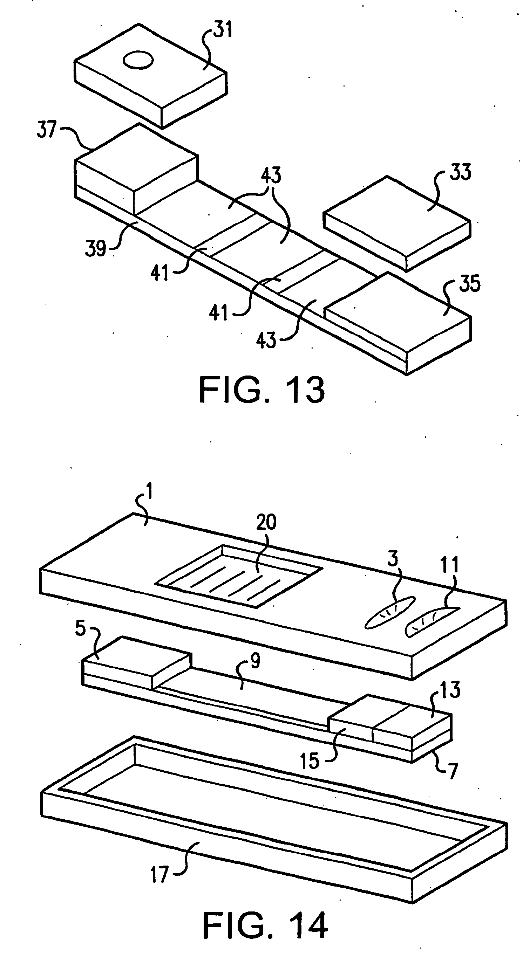

The present invention may be employed with advantage in devices of the lateral flow rate type of Figures 11-16. In one embodiment of a lateral flow device of the present invention as depicted in Figure 14, the device comprises a housing cover 1, means (port) 3 in the housing to introduce a sample to be assayed into the device, means 5 (absorbent pad) for fluid collection, and a backing strip 7 having spaced apart first and second ends. The means for sample fluid collection is adhered to the backing at a first end of the backing strip, the means to introduce the sample is adhered to the backing at the second end of the backing strip. A microporous or porous membrane 9 is optionally placed between the first and second ends to provide an avenue for travel of the sample between the first and second ends as well as to provide a matrix for any reagent material that may be present for contact with the fluid sample, during which time the sample contacts the reagent with which reaction or contact is to occur.

In accordance with the present invention, the surface of the backing strip between the first and second ends is hydrophilic in character. The backing strip 7 is heat-sealable or exhibits pressure sensitive adhesive properties. If the backing strip 7 exhibits pressure sensitive adhesive properties, the hydrophilic character of the material serves to avoid reducing the effectiveness of any membrane 9 attached to the backing strip in the event that migration of the adhesive into the membrane occurs.

By way of further advantage, due to the hydrophilic character of the backing strip, it may be possible to avoid use of the membrane 9, instead relying solely on the hydrophilic character of the backing strip itself to wick the sample from the sample introduction point to the sample collection point. In such an embodiment, the reagent with which the sample must contact or react with will either be applied directly to the backing strip for contact with the sample, or be introduced to the surface of the backing strip from a reservoir attached to the backing strip in a conventional manner.

Port 11 may be employed to provide access for another material such as a buffer to be applied to absorbent pad 13. The sample once added to port 3 contacts absorbent pad 15. The assembly of the backing strip and associated attached components may be positioned within a bottom portion 17 of the housing. The housing cover 1 includes view port 20 for viewing the visual result of the reaction between the sample and the reagent present in the device.

Figures 11 and 12 depict a lateral flow test strip according to the present invention. The test strip includes sample absorbent pad 19, membrane 21 and sample collection pad 23. Backing strip 25 includes a hydrophilic surface 27 which is heat-sealable or pressure sensitive in nature in accordance with the present invention. Areas 29 on the membrane 21 contain reagents for reaction with the sample. Alternatively, the membrane may be omitted and its function served by the hydrophilic surface of the backing strip 25. In such an embodiment, the areas 29 may still contain reagents for reaction with the test sample, and areas 29 of the backing strip may also be made more hydrophobic (or less hydrophilic) than the remaining surface of the backing strip. The presence of such areas will serve to slow the rate of passage of the sample across the backing strip to maximize time of contact with the reagents in areas 29.

Another embodiment of the device of the present invention is depicted in Figure 13. The device of Figure 13 includes covers 31,33 for the respective ends of the device, which include sample pad 37 and collection pad 35, with test zones 41 being intermediate the ends of the device on backing strip 39 having a hydrophilic surface 43. As discussed above, test zones 41 may be positioned on portions of the backing strip which have been rendered less hydrophilic (or more hydrophobic) than the remaining portion of the backing strip.

Various modifications can be undertaken with advantage in such an embodiment. As discussed above, selective areas of hydrophilic/hydrophobic surface character can be provided on the surface of the backing material to modify the flow characteristics of the fluid sample, either by directing the sample longitudinally along the backing strip toward the fluid collection point, or by causing the fluid sample to contact adjacent hydrophilic/hydrophobic areas to slow the flow rate of the fluid sample along the backing strip. In such an instance, for example, the reagent may be placed on the hydrophobic portion where the wicking of the fluid sample would be slower to permit a longer contact time with between the fluid sample and the reagent. In terms of this discussion, the term hydrophobic is not intended to mean that the portion of the backing would be entirely hydrophobic, but could also mean that that the area is more hydrophobic than the adjacent hydrophilic portion of the backing strip (i.e., both portions would have varying degrees of hydrophilicity so that the wicking of the fluid sample would still be encouraged to travel from the sample inlet to the sample collection area).

Accordingly, in the context of Figures 11-14, the surface of the backing film (e.g. a polyester film as in Figure 1) could be rendered hydrophilic by any of the methods discussed above, and employed as a heat-sealable layer for bonding to the absorbant pad and the sample pad/conjugate pad. Optionally, a membrane could also be bonded to the heat-sealable hydrophilic backing strip. Alternatively, the use of the membrane can be avoided and the reagents applied directly to the hydrophilic surface of the backing strip and the sample and reagent caused to wick directly across the surface of the backing strip toward the absorbent pad.

As discussed above, in an embodiment where the backing strip comprises a hydrophilic pressure sensitive adhesive layer, the membrane can still be used with advantage due to the hydrophilic character of the adhesive without fear of diminishment of the ability of the membrane to function due to migration of the adhesive. However, it is still possible to avoid the use of the membrane, with the hydrophilic adhesive layer serving as the transport medium for the sample from the sample pad to the absorbent pad. Any reagents desired to be contacted with the sample may be applied directly to the surface of the hydrophilic adhesive layer. The adhesive character of the backing strip can also be employed with advantage to bond the respective sample/conjugate/absorbent pads to the backing strip. This facilitates the manufacture of the device. Such a device would typically be contained in a suitable housing that generally includes a viewing window to determine the extent of the reaction of the sample and the reagent (e.g., to determine extent of reaction due to color formation or the intensity of the color formed).

It is frequently a disadvantage in any determination by means of fluorescent detection that "background" fluorescence occurs which may affect the accuracy of the desired fluorescent detection. It has previously been proposed to employ "low background" assay platforms and well plates for use in fluorescent detection methods to minimize the degree of background fluorescence during the assay. See U.S. Patent Nos. 5,910,287 and 6,171,780 in this regard. These patents teach the use of polymers having low fluorescence and high transmittance such as cycloolefins in the formation of the bottom portion of the wells in a multi-well assay platform.

It would be desirable, however, to also employ a low fluorescent sealing or cover layer either alone or in conjunction with a low fluorescent assay platform or multi-well plates to further reduce the possibility of undesirable background fluorescence during the assay by fluorescent detection.

Fluorescence is defined as "radiative transition from the lowest excited singlet state ($S_1$) to the ground state ($S_o$) (Electronic Properties of Polymers; ed. J. Mort et al, p. 177, 1982). Materials for applications where no or minimal fluorescence is preferred (such as microfluidic devices) typically have high excitation energy potential. In polymeric materials, the base monomer preferably has a high ionization potential and low electron affinity. High energy is required to excite the molecules from a ground state to an excited state. Low fluorescent compounds do not easily accept charge transfer from other compounds or excited states. Similarly, molecules that are easily polarized by the delocalization of an electron should be avoided. Aromatic compounds and compounds with a conjugated pi electron structure may be easily excited by a radiate excitation source due to their non-localized electrons.

Advantageously, such sealing or cover layers will exhibit low natural fluorescence at the excitation and detection wavelength used to detect the biomaterial; will be dimensionally stable and not flow into any microfluidic channels present; will adhere to the base plate without creating voids or gaps that may allow migration of the components from one channel to an adjacent channel; is compatible with the chemical reagents used in the reservoirs such as electrophoretic media and biomaterials including DNA fragments and polypeptides; be compatible with the separation conditions employed including pH (e.g., pH of 2-12, preferably 3-8), electric field potentials and voltage gradients of 200 volts/cm; exhibit good stability to moisture and temperature change; preferably contain no charged substituents which may interfere with the separation of biomaterials that contain charged groups; contain no leachable components that may contaminate the sample; and exhibit little or no spectral emission in the wavelength range of 400 to 800 nM. Such sealing or cover layers may possess pressure sensitive adhesive properties or be heat sealable.

With respect to the material used in the assay platform, such material may be either flexible or rigid, but is preferable that such materials be clear and colorless; chemically compatible with electrophoretic separation; exhibit little or no fluorescence under assay detection conditions as evidenced by little or no spectral emissions in the wavelength of 400 to 800 nM; be dimensionally stable and withstand pressure during electrophoresis; and dissipate heat during electrophoresis; and have minimal cross-sectional dimension.

When the sealing layer comprises a pressure sensitive adhesive or is a polymeric layer which adheres to itself, it is preferable to use a liner to protect the sealing surface. If a liner is used, it is desirable that there is no transfer of compounds from the liner to the sealing surface which will interfere with the separation of biomaterials or increase the fluorescence of the sealing surface.

Materials suitable for use in the present invention which exhibit minimal or low fluorescence and which may be used as substrate materials include but are not limited to polyolefins, polysiloxanes, polyalkylmethacrylates, and polycarbonates. Examples of suitable substrate films include Rohm PLEXIGLASS™ S30K, Rohm OROGLASS™, and Goodfellow Polymethylmethacrylate and Mitsubishi SKINKOLITE™ HBS 007.

Materials suitable for use in the present invention as sealing or cover materials which exhibit minimal or low fluorescence include but are not limited to the above materials as well as adhesives such as alkyl(meth)acrylic acid esters. Preferred adhesive compositions contain no aromatic moieties such as those found in aromatic solvents, aromatic monomers, polymerization inhibitors or polymerization initiators as the presence of the aromatic moiety will result in undesirable spectral emissions. If any aromatic solvents such as toluene or xylene are used in the formation of the adhesive, they are removed during the drying process or by subsequent treatment of the product. Silicone-based adhesives such as Dow™ 7657 or Sylgard™ 184 (polydimethyl siloxane) may also be used. A low fluorescence sealing layer may be provided with advantage comprised of amorphous polyolefins such as polyethylene, polypropylene or blends of polyolefins.

Preferred acrylate-based pressure sensitive adhesives are formulated using alkyl (alkyl)acrylate esters that are polymerized using non-aromatic initiator and cross-linkers. The concentration of unreacted components such a monomers, initiators, and crosslinkers should be minimized to ensure low fluorescence. Typically, the concentration of unreacted monomers in the formulation will be in the ppm range When an organic solvent is used in the formulation, non-aromatic solvents such as low molecular weight hydrocarbons, alcohols, and esters are preferred. Solvents such as heptane, hexane, ethyl acetate and isopropanol are Preferred. Reactive monomers such as acrylic acid may be inhibited using substituted hydroquinones. Substituted hydroquinones extend the shelf life of the reactive monomers and higher concentrations are used for the most reactive monomers. Substituted hydroquinones in the sealing layer may fluoresce significantly after exposure to radiant energy due to their low activation energy.

**Claims**

1. A lateral flow in-vitro diagnostic device comprising a housing, means in the housing to introduce a sample to be assayed in said device, means in said housing for fluid collection, and a backing strip having spaced apart first and second ends, **characterized in that** the backing strip has a surface with hydrophilic properties and comprises a hydrophilic adhesive selected from hydrophilic heat-sealable adhesives and hydrophilic pressure sensitive adhesives, wherein the adhesive increases the surface energy of a fluid flow path in the device and decreases the surface tension of a biological fluid flowing along the fluid flow path.

2. A lateral flow device of Claim 1, wherein said surface of said backing strip comprises a hydrophilic heat-sealable adhesive.

3. A lateral flow device of Claim 1, , wherein said surface of said backing strip comprises a pressure sensitive adhesive.

4. A lateral flow device of any one of Claims 1, 2 and 3, further comprising a microporous or porous membrane attached to said backing strip between said first and second ends.

5. A lateral flow device of Claim 4, wherein said microporous or porous membrane is attached to said backing strip comprises a hydrophilic pressure sensitive adhesive.

6. A lateral flow device of Claim 4, wherein said membrane is comprised of nitrocellulose.

7. A lateral flow device of any one of the preceding claims, further comprising a reagent with which said sample must contact or react with applied directly to the surface of said backing strip.

8. A lateral flow device of Claim 1 or 2, further comprising a reagent with which said sample must contact or react with contained in a reservoir attached to said backing strip.

9. A lateral flow device of any one of the preceding claims, wherein said means for fluid collection comprises an absorbent pad.

10. A lateral flow device of any one of the preceding claims , wherein said backing strip includes spaced apart hydrophilic and hydrophobic areas.

11. A lateral flow device of Claim 10, wherein a reagent with which said sample must contact or react with is applied to at least a portion of said hydrophobic areas.

12. A lateral flow device of any one of Claims 1 to 9, wherein said backing strip includes spaced apart first surface portions exhibiting hydrophilic character and second surface portions exhibiting lesser hydrophilic character than said first portions.

13. A lateral flow device of Claim 12, wherein said second areas which are less hydrophilic are formed by exposure of said polymer in said second areas to thermal radiant energy.

14. A lateral flow device of Claim 13, wherein said backing strip comprises a polymer containing an ammonium salt of nonyl phenol ethoxyl sulfonic acid.

15. A lateral flow device of Claim 12, wherein a reagent with which said sample must contact or react with is applied to at least a portion of said second portions exhibiting lesser hydrophilic character than said first portions.

16. A lateral flow device of any one of the preceding claims, wherein said backing strip includes at least one surfactant.

17. A lateral flow device of Claim 1, wherein said backing strip is comprised of a polymeric material and a surfactant is blended with said polymeric material.

18. A lateral flow device of Claim 16 or 17, wherein said surfactant is a non-ionic or anionic surfactant.

19. A lateral flow device of Claim 18, wherein said surfactant is selected from polyethylene oxide, polypropylene oxide, nonylphenol ethyoxylate and polyalkylenyeneoxide modified heptamethyltrisiloxane.

20. A lateral flow device of Claim 18, wherein said surfactant is selected from sodium or ammonium salts of nonyl phenol ethoxyl sulfonic acid, sodium lauryl sulfate, sodium 2-ethylhexyl sulfate and sodium dioctylsulfo succinate.

21. A lateral flow device of Claim 18, wherein said surfactant is selected from the ionic salt of 2-acrylamido-2-methyl propanesulfonic acid, N-vinyl caprolactam, caprolactone acrylate, N-vinyl pyrrolidone, and sulfate and acrylic monomers.

22. A lateral flow device of Claim 1, comprising a fluid transport channel which is provided with a hydrophilic surface by covalent bonding a hydrophilic surfactant to the surface of said channel.

**Patentansprüche**

1. Lateralfluss-in-vitro-Diagnosevorrichtung enthaltend ein Gehäuse, Mittel in dem Gehäuse zum Zuführen einer in der Vorrichtung zu testenden Probe, Mittel in dem Gehäuse zum Fluidsammeln, und einen Trägerstreifen mit voneinander beabstandeten ersten und zweiten Enden, **dadurch gekennzeichnet, dass** der Trägerstreifen eine Oberfläche mit hydrophilen Eigenschaften aufweist und einen hydrophilen Klebstoff enthält, der ausgewählt ist aus hydrophilen Heißsiegelklebstoffen und hydrophilen drucksensitiven Klebstoffen, wobei der Klebstoff die Oberflächenenergie eines Fluidströmungsweges in der Vorrichtung erhöht und die Oberflächenspannung eines entlang dem Fluidströmungsweg fließenden biologischen Fluids herabsetzt.

2. Lateralfluss-Vorrichtung nach Anspruch 1, wobei die Oberfläche des Trägerstreifens einen hydrophilen Heißsiegelklebstoff aufweist.

3. Lateralfluss-Vorrichtung nach Anspruch 1, wobei die Oberfläche des Trägerstreifens einen hydrophilen drucksensitiven Klebstoff aufweist.

4. Lateralfluss-Vorrichtung nach einem der Ansprüche 1, 2 und 3, die weiterhin eine mikroporöse oder poröse Membran enthält, die an dem Trägerstreifen zwischen den ersten und zweiten Enden angebracht ist.

5. Lateralfluss-Vorrichtung nach Anspruch 4, wobei die mikroporöse oder poröse Membran an dem Trägerstreifen angebracht ist, der einen hydrophilen drucksensitiven Klebstoff aufweist.

6. Lateralfluss-Vorrichtung nach Anspruch 4, wobei die Membran aus Nitrocellulose gebildet ist.

7. Lateralfluss-Vorrichtung nach einem der vorhergehenden Ansprüche, wobei sie weiterhin ein Reagenz enthält, mit dem die Probe kontaktieren oder reagieren muss, die direkt auf die Oberfläche des Trägerstreifens aufgebracht ist.

8. Lateralfluss-Vorrichtung nach Anspruch 1 oder 2, wobei sie weiterhin ein Reagenz enthält, mit dem die Probe kontaktieren oder reagieren muss, die in einem an dem Trägerstreifen angebrachten Reservoir enthalten ist.

9. Lateralfluss-Vorrichtung nach einem der vorhergehenden Ansprüche, wobei das Mittel zum Fluidsammeln ein Absorptions-Pad enthält.

10. Lateralfluss-Vorrichtung nach einem der vorhergehenden Ansprüche, wobei der Trägerstreifen voneinander beabstandet hydrophile und hydrophobe Bereiche enthält.

11. Lateralfluss-Vorrichtung nach Anspruch 10, wobei ein Reagenz, mit dem die Probe kontaktieren oder reagieren muss, zumindest auf einen Abschnitt der hydrophoben Bereiche aufgebracht ist.

12. Lateralfluss-Vorrichtung nach einem der Ansprüche 1 bis 9, wobei der Trägerstreifen voneinander beabstandet erste Oberflächenabschnitte mit hydrophilem Charakter und zweite Oberflächenabschnitte mit weniger hydrophilem Charakter als die ersten Abschnitte aufweist.

13. Lateralfluss-Vorrichtung nach Anspruch 12, wobei die zweiten Bereiche, die weniger hydrophil sind, durch Bestrahlung des Polymers in den zweiten Bereichen mit thermischer Strahlungsenergie gebildet sind.

14. Lateralfluss-Vorrichtung nach Anspruch 13, wobei der Trägerstreifen ein Polymer umfassst, das ein Ammoniumsalz einer Nonylphenolethoxylsulfonsäure enthält.

15. Lateralfluss-Vorrichtung nach Anspruch 12, wobei ein Reagenz, mit dem die Probe kontaktieren oder reagieren muss, auf zumindest einen Abschnitt der zweiten Abschnitte, die weniger hydrophilen Charakter als die ersten Abschnitte aufweisen, aufgebracht ist.

16. Lateralfluss-Vorrichtung nach einem der vorhergehenden Ansprüche, wobei der Trägerstreifen zumindest einen oberflächenaktiven Stoff enthält.

17. Lateralfluss-Vorrichtung nach Anspruch 1, wobei der Trägerstreifen aus einem Polymermaterial gebildet ist und ein oberflächenaktiver Stoff dem Polymermaterial beigemischt ist.

18. Lateralfluss-Vorrichtung nach Anspruch 16 oder 17, wobei der oberflächenaktive Stoff ein nicht-ionischer oder anionischer oberflächenaktiver Stoff ist.

19. Lateralfluss-Vorrichtung nach Anspruch 18, wobei der oberflächenaktive Stoff ausgewählt ist aus Polyethylenoxid, Polypropylenoxid, Nonylphenolethoxylat und Polyalkylenoxidmodifiziertem Heptamethyltrisiloxan.

20. Lateralfluss-Vorrichtung nach Anspruch 18, wobei der oberflächenaktive Stoff ausgewählt ist aus Natrium- oder Ammoniumsalzen von Nonylphenolethoxylsulfonsäure, Natriumlaurylsulfat, Natrium-2-Ethylhexylsulfat und Natriumdioctylsulfo succinat.

21. Lateralfluss-Vorrichtung nach Anspruch 18, wobei der oberflächenaktive Stoff ausgewählt ist aus dem ionischen Salz von 2-Acrylamido-2-methylpropansulfonsäure, N-vinylcaprolactam, Caprolactonacrylat, N-vinylpyrrolidon und Sulfat- und AcrylMonomeren.

22. Lateralfluss-Vorrichtung nach Anspruch 1, enthaltend einen Fluidtransportkanal, der mit einer hydrophilen Oberfläche durch kovalente Bindung eines hydrophilen oberflächenaktiven Stoffs an die Oberfläche des Kanals versehen ist.

**Revendications**

1. Dispositif diagnostique in vitro à écoulement latéral comprenant un logement, des moyens dans le logement pour introduire un échantillon à tester dans ledit dispositif, des moyens dans ledit logement pour collecter un fluide, et une bande de support ayant une première et un e seconde extrémités espacées, **caractérisé en ce que** la bande de support a une surface dotée de propriétés hydrophiles et comprend un adhésif hydrophile choisi parmi des adhésifs hydrophiles thermosoudables et des adhésifs hydrophiles sensibles à la pression, dans lequel l'adhésif augmente l'énergie de surface d'un chemin d'écoulement de fluide dans le dispositif et diminue la tension de surface d'un fluide biologique s'écoulant le long du chemin d'écoulement de fluide.

2. Dispositif à écoulement latéral selon la revendication 1, dans lequel ladite surface de ladite bande de support comprend un adhésif hydrophile thermosoudable.

3. Dispositif à écoulement latéral selon la revendication 1, dans lequel ladite surface de ladite bande de support comprend un adhésif sensible à la pression.

4. Dispositif à écoulement latéral selon l'une quelconque des revendications 1, 2 et 3, comprenant en outre une membrane microporeuse ou poreuse fixée à ladite bande de support entre lesdites première et seconde extrémités.

5. Dispositif à écoulement latéral selon la revendication 4, dans lequel ladite membrane microporeuse ou poreuse est fixée à ladite bande de support et comprend un adhésif hydrophile sensible à la pression.

6. Dispositif à écoulement latéral selon la revendication 4, dans lequel ladite membrane comprend de la nitrocellulose.

7. Dispositif à écoulement latéral selon l'une quelconque des revendications précédentes, comprenant en outre un réactif avec lequel ledit échantillon doit entrer en contact ou réagir appliqué directement sur la surface de ladite bande de support.

8. Dispositif à écoulement latéral selon les revendications 1 ou 2, comprenant en outre un réactif avec lequel ledit échantillon doit entrer en contact ou réagir contenu dans un réservoir fixé à ladite bande de support.

9. Dispositif à écoulement latéral selon l'une quelconque des revendications précédentes, dans lequel ledit moyen pour collecter un fluide comprend un tampon absorbant.

10. Dispositif à écoulement latéral selon l'une quelconque des revendications précédentes, dans lequel ladite bande de support comprend des zones hydrophiles et hydrophobes séparées.

11. Dispositif à écoulement latéral selon la revendication 10, dans lequel un réactif avec lequel ledit échantillon doit entrer en contact ou réagir est appliqué sur au moins une partie desdites zones hydrophobes.

12. Dispositif à écoulement latéral selon l'une quelconque des revendications 1 à 9, dans lequel ladite bande de support comprend des premières parties de surface séparées, présentant un caractère hydrophile et des secondes parties de surface présentant un caractère moins hydrophile que lesdites premières parties.

13. Dispositif à écoulement latéral selon la revendication 12, dans lequel lesdites secondes zones qui sont moins hydrophiles sont formées par exposition dudit polymère dans lesdites secondes zones à une énergie radiante thermique.

14. Dispositif à écoulement latéral selon la revendication 13, dans lequel ladite bande de support comprend un polymère contenant un sel d'ammonium de l'acide nonyl-phénol-éthoxyl sulfonique.

15. Dispositif à écoulement latéral selon la revendication 12, dans lequel un réactif avec lequel ledit échantillon doit entrer en contact ou réagir est appliqué à au moins une partie desdites secondes parties présentant un caractère moins hydrophile que lesdites premières parties.

16. Dispositif à écoulement latéral selon l'une quelconque des revendications précédentes, dans lequel ladite bande de support comprend a u moins un tensioactif.

17. Dispositif à écoulement latéral selon la revendication 1, dans lequel ladite bande de support comprend un matériau polymère et un tensioactif est mélangé audit matériau polymère.

18. Dispositif à écoulement latéral selon les revendications 16 ou 17, dans lequel ledit tensioactif est un tensioactif non-ionique ou anionique.

19. Dispositif à écoulement latéral selon la revendication 18, dans lequel ledit tensioactif est choisi parmi l'oxyde de polyéthylène, l'oxyde de polypropylène, l'éthoxylate de nonylphénol et l'heptaméthyltrisiloxane modifié en polyalkylenyeneoxide.

20. Dispositif à écoulement latéral selon la revendication 18, dans lequel ledit tensioactif est choisi parmi des sels de sodium ou d'ammonium d'acide nonyl-phénol-éthoxyl sulfonique, de laurylsulfate de sodium, de 2-éthylhexyl sulfate de sodium et de dioctylsulfo-succinate de sodium.

21. Dispositif à écoulement latéral selon la revendication 18, dans lequel ledit tensioactif est choisi parmi le sel ionique d'acide 2-acrylamido-2-méthyl propanesulfonique, le N-vinyl-caprolactame, l'acrylate de caprolactone, la N-vinyl pyrrolidone, et les monomères de sulfate et acryliques.

22. Dispositif à écoulement latéral selon la revendication 1, comprenant un canal de transport de fluide doté d'une surface hydrophile par liaison covalente d'un tensioactif hydrophile à la surface dudit canal.

FIG.1A

FIG.1B

$$h = \frac{2\,\boxed{\gamma \cos \theta}}{pgr}$$

h=HEIGHT CAPILLARY RISE
p=DENSITY OF LIQUID
g=GRAVITATION FORCE
r=RADIUS OF TUBE
$\gamma$=SURFACE TENSION OF LIQUID
$\theta$=CONTACT ANGLE

## FIG.2

$\gamma$SV=SURFACE TENSION BETWEEN SOLID AND VAPOR
$\gamma$SL=SURFACE TENSION BETWEEN SOLID AND LIQUIS
$\gamma$LV=SURFACE TENSION BETWEEN LIQUID AND VAPOR

## FIG.3

CAST LIQUID FILM  ADHESIVE POOL

FILM BACKING

COATING BARS

# FIG. 4

STAGE

HYDROPHILIC FILM

DROP

HORIZONTAL MEASURING
CROSS-LINE

ROTATE VERTICLE MEASURING
CROSS-LINE TO TANGENCY

VERTICLE MEASURING
CROSS-LINE

# FIG. 5

FIG. 6

FIG. 11

FIG. 12

FIG. 7

EP 1 390 750 B1

FIG. 8

EP 1 390 750 B1

FIG. 9

FIG. 10

FIG. 13

FIG. 14

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 5712172 A
- US 5804452 A
- US 4883642 A
- US 579827 A
- EP 833159 A
- WO 9738126 A
- US 5916521 A
- WO 9934191 A
- US 4857453 A
- US 5087556 A
- US 5137808 A
- US 5712170 A
- US 5821073 A
- US 5985675 A
- US 5989921 A
- US 6087175 A
- US 6103536 A
- US 6048498 A
- US 6117395 A
- US 3686355 A
- US 5354815 A
- US 5614598 A
- US 5508313 A
- US 5660178 A
- US 6121508 A
- WO 0056828 A
- EP 869979 B
- US 5685758 A
- WO 9748779 A
- US 6040048 A
- US 2502841 A
- US 2829070 A
- US 3142582 A
- US 3326742 A
- US 3561995 A
- US 3843617 A
- US 3968309 A
- US 4190689 A
- US 4387183 A
- US 4416749 A
- US 4460652 A
- US 4595632 A
- US 4666452 A
- US 5273812 A
- US 5280084 A
- US 5332625 A
- US 5451460 A
- US 5503897 A
- US 5910287 A
- US 6171780 A

### Non-patent literature cited in the description

- **S.M. Rosen.** Biomarkers of chemical exposure: A new Frontier in Clinical Chemistry. *IVD Technology,* May 1996, 22
- **RA. Esposito ; A.T. Culliford ; S.B. Colvin et al.** The Role of the Activated Clotting Time in Herparin Administration and Neutralization for Cardiopulmonary Bypass. *J. Thor. Card. Surg.,* 1983, vol. 85, 174-185
- **C.A. McDonald ; P. Syribeys ; B. Hazelton ; P. Bethea ; T. Rigl ; S. Hydo ; S.J. Kennedy.** A rapid 1-step colored particle lateral flow immunoassay for the detection of Group 1 Streptococcal Antigen extracted directly from Throat Swats. *93rd General Meeting of American Society Microbiology,* 1993, vol. 93, 507
- Glucose Monitor without Fingersticking. IVD Technology. Amira Medical, July 1999, 16
- **Jones et al.** *IVD Technology,* September 2000, 57-63
- **N. Vallespi i Salvado et al.** Surfactants in Pressure Sensitive Adhesives. *Surface Coatings International,* 1999, vol. 4, 181-185
- **Walter J. Moore.** Physical Chemistry. Prentice-Hall, 1962, 730
- Influence of Constitution on Adhesion. **W.A. Zisman.** Handbook of Adhesives. Van Nostrand Reinhold Co, 1977, 38
- **T. Young.** *Philos. Trans. Roy. Soc. London,* vol. 95, 65
- **Harkins.** The Physical Chemistry of Surface Films. Reinhold, 1952
- **M.J. Rosen.** Surfactant and Interfacial Phenomena. John Wiley & Sons, 1978
- **Th.F. Tadros.** Surfactants. Academic Press, Inc, 1984
- **A.C. Clark et al.** New and Improved Waterborne Systems. *Adhesives Age,* September 1999, 33-40

- **A. Doring et al.** Atomic Force Microscopy: Micro- and Nano-Mapping of Adhesion, Tack and Viscosity. *23rd Annual Technical Seminar: Pressure Sensitive Adhesive Tapes for the New Millennium,* May 2000, 213-222

- Influence of Constitution on Adhesion. **W.A. Zisman.** Handbook of Adhesives. 1977, 46
- Electronic Properties of Polymers. 1982, 177